(19) Europäisches Patentamt
European Patent Office
Office européen des brevets

(11) Veröffentlichungsnummer: **0 095 636**
**A1**

(12) **EUROPÄISCHE PATENTANMELDUNG**

(21) Anmeldenummer: **83104787.3**

(22) Anmeldetag: **16.05.83**

(51) Int. Cl.³: **C 07 C 131/105**, C 07 C 119/16, A 01 N 53/00, A 01 N 37/52, C 07 C 131/00

(30) Priorität: **28.05.82 DE 3220106**

(43) Veröffentlichungstag der Anmeldung: **07.12.83 Patentblatt 83/49**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Führer, Wolfgang, Dr., Wehrstrasse 28, D-5202 Hennef 1 (DE)**
Erfinder: **Stetter, Jörg, Dr., Gellertweg 4, D-5600 Wuppertal 1 (DE)**
Erfinder: **Naumann, Klaus, Dr., Richard-Wagner-Strasse 6, D-5090 Leverkusen 1 (DE)**
Erfinder: **Hammann, Ingeborg, Dr., Lutherstrasse 22, D-4330 Mülheim/Ruhr (DE)**

(54) **N-substituierte Iminoester, Verfahren zu ihrer Herstellung sowie ihre Verwendung in Schädlingsbekämpfungsmitteln.**

(57) Die vorliegende Erfindung betrifft neue N-substituierte Iminoester der Formel (I)

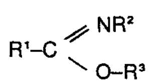 (I)

in welcher

$R^1$ für den Rest einer bei Pyrethroiden verwendbaren Säure steht,

$R^2$ für Alkoxy, Amino, Alkylamino, Dialkylamino oder einem über Stickstoff gebundenen stickstoffhaltigen Heterocyclus steht,

$R^3$ für den Rest eines bei Pyrethroiden verwendbaren Alkohols steht,

sowie neue Zwischenprodukte zu ihrer Herstellung.

Man erhält sie, indem man Halogenide der Formel (II)

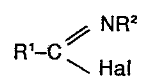 (II)

in welcher

$R^1$ und $R^2$ die vorstehend genannte Bedeutung besitzen und

Hal für Halogen steht, mit Alkoholen der Formel (III)

$$HO-R^3 \qquad (III)$$

in welcher

$R^3$ die obengenannte Bedeutung besitzt, umsetzt.

Die neuen N-substituierten Iminoester der Formel (I) weisen hervorragende insektizide und akarizide Eigenschaften bei günstiger Fischtoxizität auf.

EP 0 095 636 A1

 0095636

BAYER AKTIENGESELLSCHAFT 5090 Leverkusen, Bayerwerk

Zentralbereich
Patente, Marken und Lizenzen Rt/bc/c

# N-substituierte Iminoester, Verfahren zu ihrer Herstellung sowie ihre Verwendung in Schädlingsbekämpfungsmitteln

Die Erfindung betrifft neue N-substituierte Iminoester, ein Verfahren zu ihrer Herstellung sowie ihre Verwendung in Schädlingsbekämpfungsmitteln, insbesondere in Insektiziden und Akariziden.

Ester von substituierten Cyclopropancarbonsäuren oder Phenylessigsäuren, beispielsweise der Ester der 2,2-Dimethyl-3-(2',2'-dimethyl-vinyl)-cyclopropancarbonsäure mit N-Hydroxymethylcyclohex-1-en-1,2-dicarbonsäureimid (Neopynanin, vgl. japanische Patentschrift 10 895/66) sind als Insektizide und Akarizide vom Pyrethroid-Typ bekannt. Dabei zeichnen sich diese Verbindungen durch günstige Werte der Fischtoxizität aus.

Die zufriedenstellende Wirkung dieser Verbindungen ist allerdings bei geringen Wirkstoffkonzentrationen und Aufwandmengen nicht in jedem Fall gewährleistet.

Es wurden nun neue N-substituierte Iminoester der Formel (I)

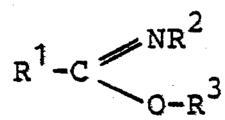

(I)

gefunden, in welcher

$R^1$ für den Rest einer bei Pyrethroiden verwendbaren Säure steht,

$R^2$ für Alkoxy, Amino, Alkylamino, Dialkylamino oder einen über Stickstoff gebundenen stickstoffhaltigen Heterocyclus steht,

$R^3$ für den Rest eines bei Pyrethroiden verwendbaren Alkohols steht.

Die Verbindungen der Formel (I) können im Bereich des Iminoester-Strukturelementes in der syn- oder anti-Form vorliegen. Vorwiegend fallen sie als Gemische beider Formen an. Daneben können die Reste der bei Pyrethroiden verwendbaren Säuren und Alkohole (vgl. auch R. Weyler, Chemie der Pflanzenschutz- und Schädlingsbekämpfungsmittel, Band 7, Springer-Verlag 1981, Seite 92 ff) weitere geometrische und optische Isomeriemöglichkeiten bieten. Die Erfindung betrifft sowohl die geometrischen als auch die optischen Isomeren und deren Gemische.

Weiterhin wurde gefunden, daß man die neuen N-substituierten Iminoester der Formel (I) erhält, indem man Halogenide der Formel (II)

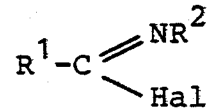

(II)

in welcher
$R^1$ und $R^2$ die vorstehend genannte Bedeutung besitzen und
Hal für Halogen steht,

mit Alkoholen der Formel

$$HO-R^3 \qquad (III)$$

in welcher
$R^3$ die obengenannte Bedeutung besitzt

oder mit einem reaktionsfähigen Derivat dieser Alkohole gegebenenfalls in Gegenwart eines säurebindenden Mittels und gegebenenfalls in Gegenwart eines Verdünnungsmittels sowie gegebenenfalls in Gegenwart eines Phasentransfer-Katalysators umsetzt.

Weiterhin wurden die Halogenide der Formel (II) gefunden

$$R^1-\underset{\displaystyle Hal}{\underset{|}{C}}=N-R^2 \qquad (II)$$

in welcher
$R^1$ für den Rest einer bei Pyrethroiden verwendbaren Säure steht,
$R^2$ für Alkoxy, Amino, Alkylamino, Dialkylamino oder einen über Stickstoff gebundenen stickstoffhaltigen Heterocyclus steht,
Hal für Halogen steht.

Weiterhin wurde gefunden, daß man die Halogenide der Formel (II) erhält, indem man stickstoffsubstituierte Carbonsäureamide, für die die beiden tautomeren Formeln (IVa) und (IVb) zur Beschreibung dienen können,

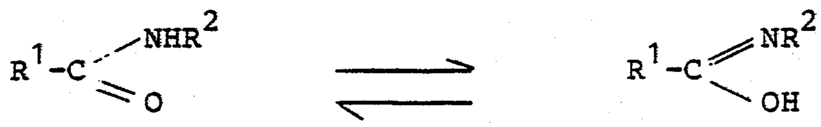

(IVa)
(IVb)

in welchen
$R^1$ und $R^2$ die obengenannten Bedeutungen besitzen,

mit bekannten Halogenierungsmitteln gegebenenfalls in Gegenwart eines Katalysators und gegebenenfalls in Gegenwart eines Säureakzeptors sowie gegebenenfalls in Gegenwart eines Verdünnungsmittels umsetzt.

Schließlich wurde gefunden, daß die neuen stickstoffsubstituierten Iminoester der Formel (I) hervorragende insektizide und akarizide Eigenschaften bei günstiger Fischtoxizität aufweisen.

Die erfindungsgemäßen neuen Iminoester mit Stickstoffsubstitution sind durch die Formel (I) allgemein definiert. In dieser Formel stehen vorzugsweise

$R^1$ für die Gruppierung

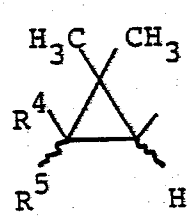

in welcher

$R^4$ für Alkyl, Alkenyl oder Alkinyl mit jeweils bis zu 6 C-Atomen, die gegebenenfalls einen carbocyclischen Ring enthalten und die gegebenenfalls substituiert sind durch ein oder mehrere Halogenatome, insbesondere Fluor, Chlor oder Brom, die außerdem gegebenenfalls substituiert sind durch einen Phenylrest, der gegebenenfalls durch Halogen, insbesondere Fluor, Chlor oder Brom substituiert ist, steht und

$R^5$ für Wasserstoff oder Methyl steht

oder für die Gruppierung

$$\begin{array}{r} R^7 \\ | \\ R^6-CH- \end{array}$$

in welcher

$R^6$ für Phenyl steht, das gegebenenfalls substituiert ist durch $C_1$- bis $C_6$-Alkyl, Halogenalkyl mit 1 bis 2 C-Atomen und bis zu 5 Halogenatomen, insbesondere Fluor, Chlor oder Brom, Halogenalkoxy mit 1 bis 2 C-Atomen und bis zu 5 Halogenatomen, insbesondere Fluor, Chlor oder Brom, Methylendioxy oder Ethylendioxy, die jeweils gegebenenfalls durch Halogen, insbesondere Fluor, Chlor oder Brom substituiert sind und

$R^7$ für $C_2$- bis $C_4$-Alkyl, bevorzugt Isopropyl, steht,

$R^2$ für $C_1$- bis $C_4$-Alkoxy, Amino, $C_1$- bis $C_4$-Alkylamino, $C_1$- bis $C_4$-Dialkylamino oder einen über Stickstoff gebundenen Morpholino-, Pyrolidino- oder Piperidino-Rest steht und

$R^3$ für Benzyl, das in α-Stellung gegebenenfalls durch CN, $-C\equiv CH$, $-C=C=CH_2$, substituiert ist und das im Kern gegebenenfalls durch Halogen, insbesondere Fluor, Chlor oder Brom oder Phenoxy, Benzyl oder Benzyloxy, die gegebenenfalls durch Halogen, wie Fluor, Chlor oder Brom substituiert sind, steht.

Besonders bevorzugt sind die Reste Pentafluorbenzyl und 3-Phenoxybenzyl, das gegebenenfalls durch Fluor und in α-Stellung durch CN substituiert ist.

Als erfindungsgemäße Stoffe der Formel (I) seien, ohne die Erfindung dadurch einzuschränken, beispielsweise genannt:

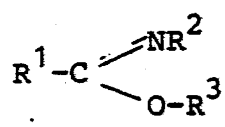 (I)

| $R^1$ | $R^2$ | $R^3$ |
|---|---|---|
| 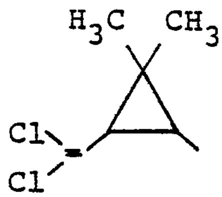 | $-OCH_3$ | 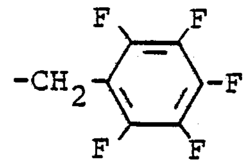 |
| " | $-OCH_3$ | 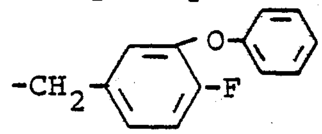 |
| " | $-N(CH_3)_2$ | 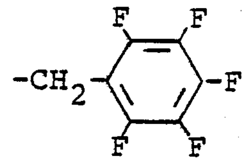 |
| 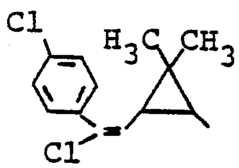 | $-N(CH_3)_2$ | 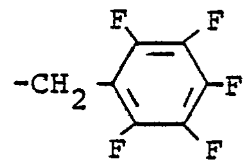 |
| 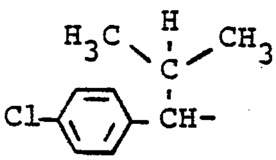 | $-OCH_3$ | 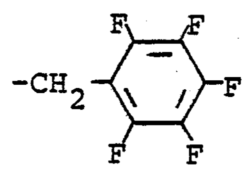 |
| 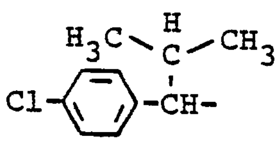 | $-N(CH_3)_2$ | 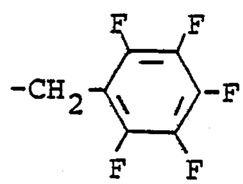 |
| 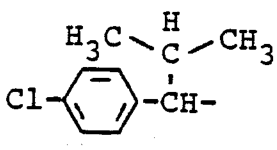 | $-OCH_3$ | 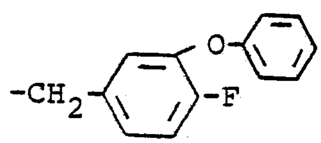 |
| 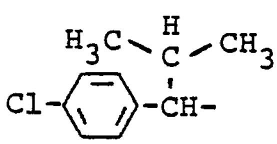 | $-N(CH_3)_2$ | 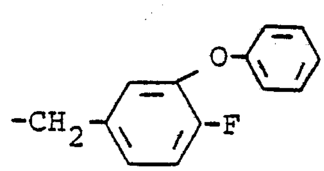 |

| $R^1$ | $R^2$ | $R^3$ |
|---|---|---|
| 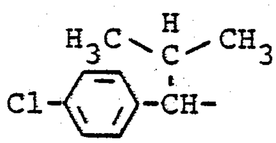 | $-OCH_3$ | 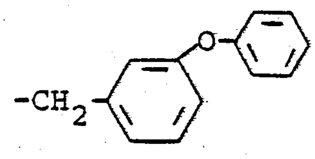 |
| 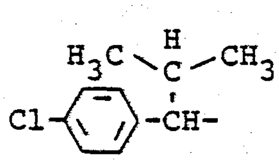 | $-N(CH_3)_2$ | 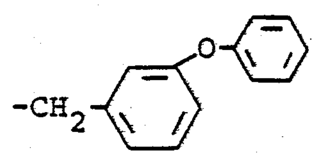 |
| 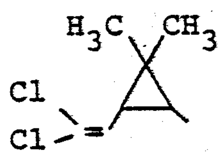 | $-N(CH_3)_2$ | 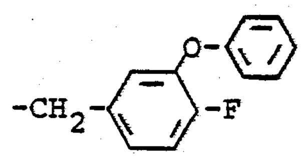 |
| 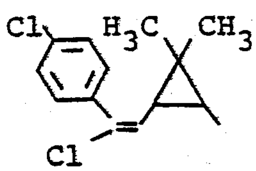 | $-OCH_3$ | 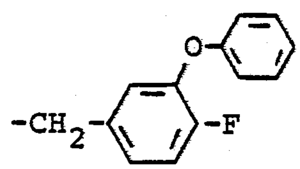 |
| 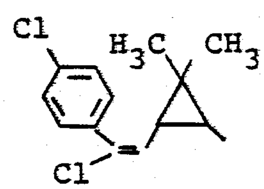 | $-N(CH_3)_2$ | 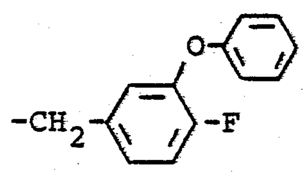 |
| 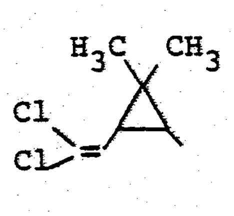 | $-OCH_3$ | 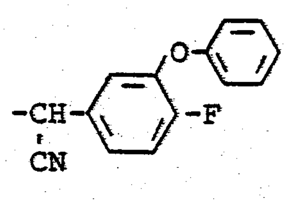 |

Verwendet man beispielsweise 1-Chlor-1-methoximino-2-(p-chlorphenyl)-3-methylbutan und 3-Benzyl-benzylalkohol als Ausgangsstoffe, so läßt sich der Reaktionsverlauf des erfindungsgemäßen Verfahrens durch das nachstehende Formelschema beschreiben:

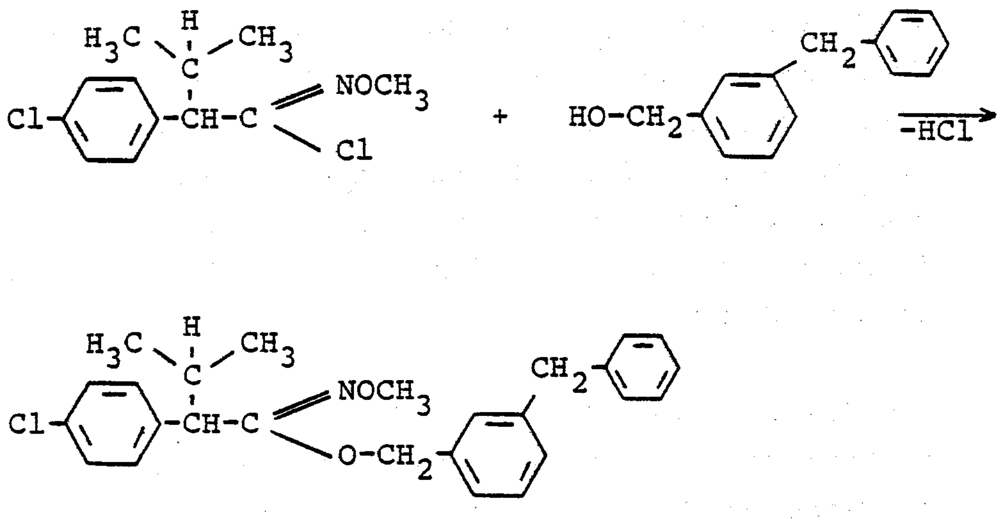


Die bei der Durchführung des erfindungsgemäßen Verfahrens als Ausgangsstoffe benötigten Halogenverbindungen sind durch die Formel (II) allgemein definiert. In dieser Formel stehen $R^1$ und $R^2$ vorzugsweise für diejenigen Reste, die bereits im Zusammenhang mit den erfindungsgemäßen Stoffen der Formel (I) vorzugsweise für diese Reste als Bedeutung genannt wurden.

Die für die Durchführung des erfindungsgemäßen Verfahrens ebenfalls erforderlichen Alkohole sind durch die Formel (III) allgemein definiert. In dieser Formel steht $R^3$ vorzugsweise ebenfalls für die Bedeutung derjenigen

Reste, die bereits in Zusammenhang mit den erfindungsgemäßen Stoffen der Formel (I) vorzugsweise für die Bedeutung dieser Reste genannt wurde.

Als reaktionsfähige Derivate der Alkohole der Formel (III) sind vorzugsweise deren Alkoholate, insbesondere Natrium-, Kalium-, Calcium-, Magnesium- oder Aluminiumalkoholate und für den Fall einer Cyansubstitution in der Benzylstellung die entsprechenden Aldehyde zu verstehen, die in Gegenwart von Alkalicyaniden ohne Zwischenisolierung des entsprechenden α-Cyanoalkohols eingesetzt werden können.

Die Alkohole der Formel (III) sowie deren reaktionsfähige Derivate sind bereits bekannt und können nach beschriebenen Verfahren erhalten werden (vgl. z.B. R. Wegler, Chemie der Pflanzenschutz- und Schädlingsbekämpfungsmittel, Band 7, Springer-Verlag, 1981, Seite 91 ff, dort weitere Originalliteratur).

Bei der Durchführung des erfindungsgemäßen Verfahrens kommen als säurebindende Mittel alle gebräuchlichen Säureakzeptoren in Betracht, beispielsweise anorganische Basen, wie Natriumhydroxid, Kaliumhydroxid, Calciumhydroxid, oder Oxide, wie Calciumoxid oder Magnesiumoxid, ferner anorganische Salze, wie Soda, Pottasche oder Kalk, aber auch organische Basen, vorzugsweise Stickstoffverbindungen wie Amine, beispielsweise Triethylamin, Cyclohexylamin, Anilin, Dimethylanilin, oder heterocyclische Stickstoffbasen, wie Pyridin,Lutidin, Collidin, Chinolin, Pyrrolidin, Piperidin, Morpholin,

Diazabicyclooctan, Diazabicyclononen oder Diazabicycloundecen.

Das erfindungsgemäße Verfahren wird gegebenenfalls in einem Verdünnungsmittel durchgeführt. Dazu können praktisch alle inerten organischen Solventien, gegebenenfalls auch Wasser, verwendet werden. Bevorzugt ist auch das Arbeiten in einem zweiphasigen System unter Verwendung eines Phasentransferkatalysators. Vorzugsweise kommen als organische Lösungsmittel in Betracht aliphatische oder aromatische, gegebenenfalls halogenierte Kohlenwasserstoffe, wie Pentan, Hexan, Cyclohexan, Heptan, Petrolether, Benzin, Ligroin, Benzol, Toluol, Xylol, Dichlormethan, Dichlorethan, Chloroform, Tetrachlorkohlenstoff, Chlorbenzol und Dichlorbenzol, ferner Ether, wie Diethylether, Dibutylether, Diisopropylether, Glykoldimethylether, Diglykoldimethylether, Tetrahydrofuran und Dioxan, ebenso Ketone, wie Aceton, Methylethylketon, Methylisopropylketon oder Methylisobutylketon, außerdem Ester, wie Essigsäuremethyl- oder Essigsäureethylester, oder Nitrile, wie beispielsweise Acetonitril oder Propionsäurenitril, im weiteren auch Amide, wie z.B. Dimethylformamid, Dimethylacetamid oder N-Methylpyrrolidon, sowie Solventien wie Dimethylsulfoxid, Tetramethylensulfon oder Hexamethylphosphorsäuretrianid.

Wird das erfindungsgemäße Verfahren in einem zweiphasigen Lösungsmittelgemisch durchgeführt, vorzugsweise in Wasser und einem der vorstehend genannten Solventien, soweit diese mit Wasser nicht oder nur begrenzt

mischbar sind, so ist die Verwendung eines Phasentransferkatalysators bevorzugt. Als solche können insbesondere quartäre Ammoniumverbindungen eingesetzt werden, wie beispielsweise Triethylbenzylammoniumbromid, oder Kronenether, wie 18-Krone-6.

Das erfindungsgemäße Verfahren läßt sich innerhalb eines größeren Temperaturbereiches durchführen. Man arbeitet im allgemeinen zwischen -40 und +160°C, vorzugsweise zwischen 0 und 120°C.

Das erfindungsgemäße Verfahren wird im allgemeinen bei Normaldruck durchgeführt.

Die Ausgangsstoffe der Formel (II) und (III) werden in der Regel in etwa äquimolaren Mengen eingesetzt. Ein Überschuß der einen oder der anderen Komponente ist möglich. Die Umsetzung wird im allgemeinen in einem geeigneten Verdünnungsmittel durchgeführt. Bei Zugabe eines Säureakzeptors kann dieser äquimolar oder auch im Überschuß dosiert werden, vorzugsweise wird er äquimolar bis zum doppelten Überschuß eingesetzt. Wird die Umsetzung in einem zweiphasigen Gemisch durchgeführt, so kann der gegebenenfalls ebenfalls zu verwendende Phasentransferkatalysator bis zur äquimolaren Menge eingesetzt werden, vorzugsweise zwischen 0,5 % und 50 %. Das Reaktionsgemisch wird im allgemeinen mehrere Stunden bei der erforderlichen Temperatur gerührt. Die Aufarbeitung des Reaktionsansatzes erfolgt nach den üblichen Methoden (s. hierzu auch die experimentellen Beispiele).

Die neuen Verbindungen der Formel (I) fallen zum Teil in Form von Ölen an, die nicht in jedem Fall unzersetzt destilliert werden können. Sie können jedoch durch längeres Aufheizen im Vakuum auf mäßig erhöhte Temperaturen von flüchtigen Anteilen befreit und auf diese Weise gereinigt werden. Ebenso lassen sich die allgemein bekannten Methoden der präparativen Chromatographie zur Reinigung heranziehen.

Zur Charakterisierung der neuen Stoffe dient der Siedepunkt, soweit eine Destillation möglich ist, sonst der Brechungsindex. Soweit die neuen Verbindungen in fester Form anfallen, können sie durch Umkristallisieren gereinigt werden. In diesem Fall dient zur Charakterisierung der Schmelzpunkt.

Die bei der Durchführung des erfindungsgemäßen Verfahrens als Ausgangsstoffe erforderlichen Halogenverbindungen der Formel (II) sind neu. Sie lassen sich in an sich bekannter Weise herstellen (vgl. Houben-Weyl, Methoden der Organischen Chemie, Band 7, Seite 692; bzw. W. Lossen, Ber. Dtsch. Chem. Ges. 18, 1189, (1885)), indem man beispielsweise N-Ethoxy-2-(p-chlorphenyl)-3-Methyl-buttersäureamid mit Phosphorpentachlorid halogeniert. Für diesen Fall kann die entsprechende Umsetzung durch das nachstehende Formelschema beschrieben werden:

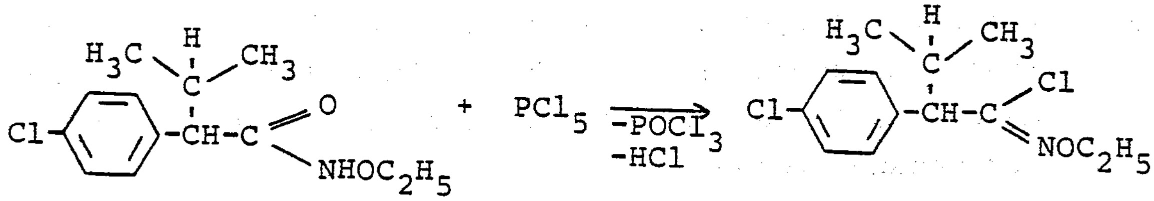

Die bei der Durchführung dieser Reaktion erforderlichen, als Ausgangsstoffe dienenden stickstoffsubstituierten Carbonsäureamide sind durch die Formeln (IVa) bzw. (IVb) in Form ihrer Tautomeren allgemein definiert. In diesen beiden Formeln besitzen $R^1$ und $R^2$ vorzugsweise diejenigen Bedeutungen, die bereits im Zusammenhang mit der Beschreibung der erfindungsgemäßen Stoffe der Formel (I) vorzugsweise für seine Reste genannt wurden.

Sie können in einfacher Weise nach allgemein üblichen Verfahren erhalten werden, indem man die entsprechenden Carbonsäuren oder deren reaktive Derivate, wie Ester oder Halogenide, mit entsprechenden Stickstoffverbindungen umsetzt. Die entsprechenden Carbonsäuren und deren Derivate sind bekannt (vgl. R. Wegler, s.o., Seite 91 ff und die dort zitierte Originalliteratur).

Als Halogenierungsmittel für die Verbindungen der Formel (IVa) bzw. (IVb) kommen vorzugsweise die gängigen Chlorüberträger in Betracht, beispielsweise seien genannt Phosphorpentachlorid, Phosphoroxychlorid, Phosphortrichlorid, Thionylchlorid oder Phosgen, Oxalylchlorid.

Als Katalysator bei dieser Halogenierung kann vorzugsweise eine stickstoffhaltige Verbindung, beispielsweise Dimethylformamid oder N-Methylformanilid benutzt werden.

Als Säureakzeptor werden bei dieser Umsetzung vorzugsweise diejenigen säurebindenden Mittel eingesetzt, die schon vorstehend für die Herstellung der erfindungsgemäßen Stoffe der Formel (I) vorzugsweise als Säurebinder genannt werden.

Als Verdünnungsmittel können bei dieser Reaktion alle inerten organischen Solventien benutzt werden, wobei vorzugsweise in Betracht kommen aliphatische und aromatische, gegebenenfalls halogenierte Kohlenwasserstoffe, wie Pentan, Hexan, Heptan, Petrolether, Benzin, Ligroin, Petroleum, Benzol, Toluol, Xylol, Dichlormethan, Dichlorethan, Trichlorethylen, Chloroform, Tetrachlorkohlenstoff, Chlorbenzol und Dichlorbenzol, und Ether, wie Diethylether, Dibutylether oder Glykoldimethylether.

Die Halogenierung kann in einem größeren Temperaturbereich durchgeführt werden. Im allgemeinen arbeitet man bei Temperaturen zwischen -20 und 150°C, vorzugsweise zwischen 0 und 100°C. Die Reaktion wird im allgemeinen bei Normaldruck durchgeführt.

Die stickstoffsubstituierten Carbonsäureamide der Formel (IVa) bzw. (IVb) und das Halogenierungsmittel werden in der Regel in äquimolaren Mengen eingesetzt. Ein Überschuß des Halogenierungsmittels ist möglich, wobei das überschüssige Halogenierungsmittel gleichzeitig als Verdünnungsmittel dienen kann. Der Katalysator wird vorzugsweise in Mengen zwischen 0,5 und 30 % eingesetzt, der Säureakzeptor äquimolar bis zum doppelten Überschuß. Das Reaktionsgemisch wird im allgemeinen mehrere Stunden bei der erforderlichen Temperatur gerührt, danach wird es nach den üblichen Verfahren aufgearbeitet.

Die neuen Halogenverbindungen der Formel (II) fallen zum Teil in Form von Ölen an. Sie sind teilweise nur

wenig stabil und müssen direkt nach dem erfindungsgemäßen Verfahren zur Herstellung der Stoffe der Formel (I) weiterverarbeitet werden. Eine Destillation ist nicht in jedem Fall möglich. Längeres vorsichtiges Aufheizen auf mäßige Temperaturen im Vakuum gestattet allerdings ein Entfernen der flüchtigen Verunreinigungen. Zur Charakterisierung kann dann das Infrarotspektrum der Verbindung dienen, das die Schwingungen der N-H- und C=O-Bindungen der Ausgangsstoffe der Formel (IVa) ebensowenig wie eine OH-Schwingung aus der tautomeren Formel (IVb) zeigen darf. Zur Charakterisierung dient auch der Brechungsindex, im Falle destillierbarer Substanzen der Siedepunkt und im Falle kristallisierender Verbindungen nach Umkristallisation der Schmelzpunkt.

Die stickstoffsubstituierten Carbonsäureamide der Formel (IVa) bzw. (IVb) werden vorzugsweise erhalten, indem man das Chlorid einer bei Pyrethroiden verwendbaren Säure der Formel

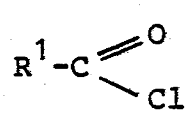 (V)

mit Stickstoffverbindungen der Formel

$$H_2N-R^2 \quad \text{(VI)}$$

gegebenenfalls in Gegenwart eines Säureakzeptors wie Triethylamin oder Pyridin und eines Verdünnungsmittels wie Toluol oder Tetrahydrofuran zwischen 0 und 80°C

umsetzt. In diesen beiden Formeln besitzen $R^1$ und $R^2$ die vorstehend genannten Bedeutungen. Die Verbindungen der Formel (V) sind bekannt (vgl. R. Wegler, s.o.), die Verbindunen der Formel (VI) sind ebenfalls allgemein bekannt.

0095636

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

0095636

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

0095636

Herstellungsbeispiele

1.)

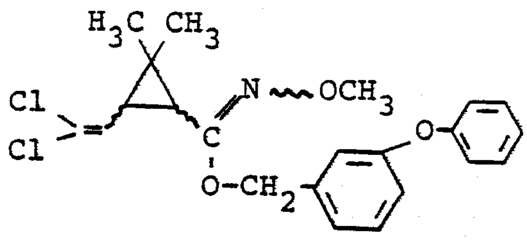

$C_{22}H_{23}Cl_2NO_3$

Zu einer Suspension von 1,5 g (0,05 Mol) Natriumhydrid (80 %ig in Öl) und 100 ml Toluol werden 8 g (0,04 Mol) 3-Phenoxybenzylalkohol gegeben. Nach Abklingen der $H_2$-Entwicklung werden dann bei 110°C und Feuchtigkeitsausschluß 10 g (0,039 Mol) (±)-cis/trans-syn/anti-Isomerengemisch, 1-Methoximinochlormethyl-2,2-dimethyl-3-2',2'-dichlorvinylcyclopropan, gelöst in 50 ml Toluol, eingetropft, drei Stunden bei gleicher Temperatur weiter gerührt und nach Stehen über Nacht, Waschen mit Wasser und Einengen im Kugelrohr destilliert. Bei 210 bis 230 0/$_{0.7}$ ergeben sich 7,7 g (47 %) eines hellen Öls vom $n_D^{20}$ = 1.6021.

Analog lassen sich erhalten:

2.)

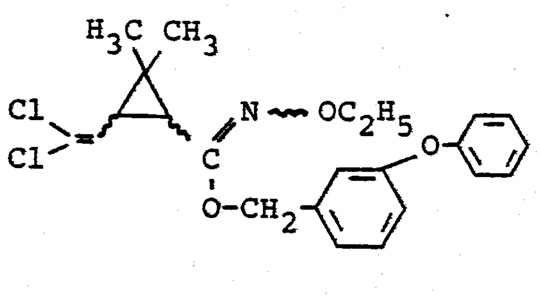

$C_{23}H_{25}Cl_2NO_3$
Sdp. 200/0.3
(Kugelrohr)
$n_D^{20}$ = 1.5262

3.) 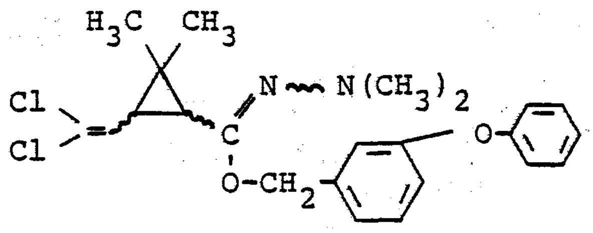

$C_{23}H_{26}Cl_2N_2O_2$
chromatographisch an Kieselgel gereinigt
$n_D^{20} = 1.5633$

Herstellung der Vorstufen:

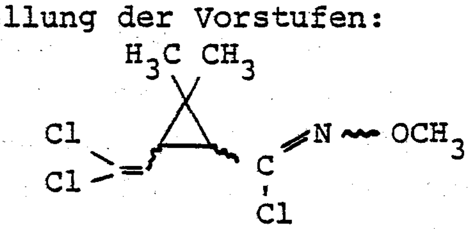

$C_9H_{12}Cl_3NO$

20 g (0,084 Mol) 2,2-Dimethyl-3-2',2'-dichlorvinyl-cyclopropancarbonsäure-N-methoxyamid ((±)-cis/trans-Isomerengemisch) werden mit 17,5 g (0,084 Mol) Phosphorpentachlorid gemischt und nach Abklingen der HCl-Entwicklung noch 30 Minuten auf 80°C erwärmt. Nach zweimaligem Einengen mit je 100 ml Toluol im Vakuum wird das verbleibende Öl im Kugelrohr destilliert, wobei bei 130 $^0/_{0.4}$ 17,5 g (81,2 %) eines hellen Öls mit dem $n_D^{20} = 1.5600$ übergehen.

Analog lassen sich erhalten:

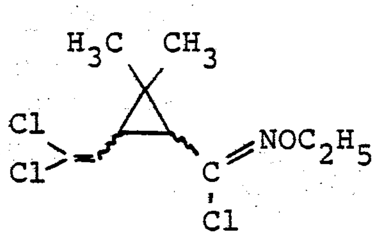

$C_{10}H_{14}Cl_3NO$
Sdp. 1040/0.4
$n_D^{20} = 1.5051$

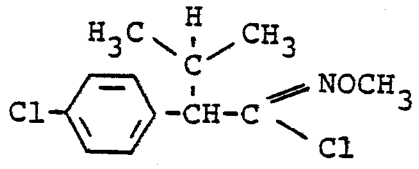

$C_{12}H_{15}Cl_2NO$
Sdp. 140/0.7
$n_D^{20} = 1.5261$

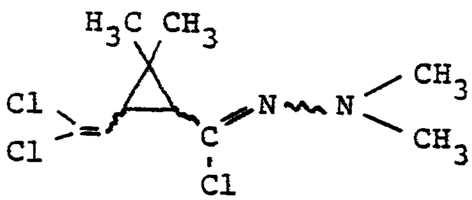

$C_{10}H_{15}Cl_3N_2$
rötliches Öl, wenig stabil, wurde sofort weiter umgesetzt

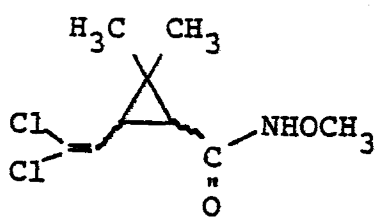

$C_9H_{13}Cl_2NO_2$

Zu einer 1 Stunde bei Raumtemperatur gerührten Suspension von 25 g (0,3 Mol) O-Methylhydroxylamin-Hydrochlorid, 400 ml Toluol und 51 g (0,5 Mol) Triethylamin werden bei 0°C 45,5 g 2,2-Dimethyl-3-2',2'-dichlorvinylcyclopropancarbonsäurechlorid (Isomerengemisch), in 100 ml Toluol gelöst, zugetropft. Nach Stehen über Nacht, Waschen mit Wasser, Trocknen mit Natriumsulfat und Einengen im Vakuum verbleibt ein helles Öl (quantitative Ausbeute, $n_D^{20} = 1.5197$), das bei längerem Stehen halbfest erstarrt.

Analog können erhalten werden:

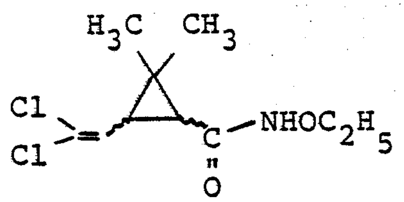

$C_{10}H_{15}Cl_2NO_2$
$n_D^{20}$ = 1.5100

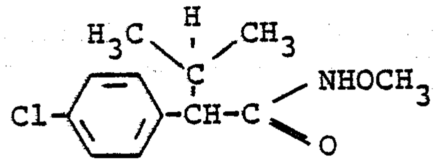

$C_{12}H_{16}ClNO_2$
Sdp. 120/0.6 (Kugelrohr)

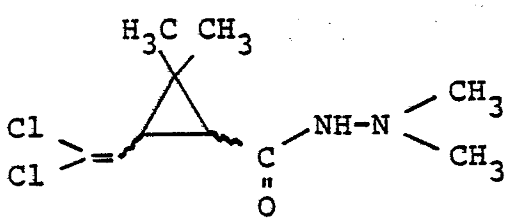

$C_{10}H_{16}Cl_2N_2O$
viskose, teilkristalline Masse
Fp. 111-115°C (von Öl abgepreßt)

Beispiel A

Laphygma-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 3

Beispiel B

Tetranychus-Test (resistent)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 3

Patentansprüche

1) N-substituierte Iminoester der Formel (I)

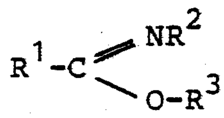

(I)

in welcher

$R^1$ für den Rest einer bei Pyrethroiden verwendbaren Säure steht,

$R^2$ für Alkoxy, Amino, Alkylamino, Dialkylamino oder einen über Stickstoff gebundenen stickstoffhaltigen Heterocyclus steht,

$R^3$ für den Rest eines bei Pyrethroiden verwendbaren Alkohols steht.

2) Verfahren zur Herstellung der N-substituierten Iminoester der Formel (I)

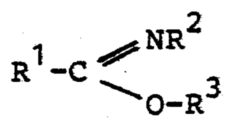

(I)

in welcher

$R^1$ für den Rest einer bei Pyrethroiden verwendbaren Säure steht,

$R^2$ für Alkoxy, Amino, Alkylamino, Dialkylamino oder einen über Stickstoff gebundenen stickstoffhaltigen Heterocyclus steht,

$R^3$ für den Rest eines bei Pyrethroiden verwendbaren Alkohols steht,

indem man Halogenide der Formel (II)

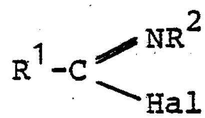

(II)

in welcher

$R^1$ und $R^2$ die vorstehend genannte Bedeutung besitzen und

Hal für Halogen steht,

mit Alkoholen der Formel

$$HO\text{-}R^3 \qquad \text{(III)}$$

in welcher

$R^3$ die obengenannte Bedeutung besitzt

oder mit einem reaktionsfähigen Derivat dieser Alkohole, gegebenenfalls in Gegenwart eines säurebindenden Mittels und gegebenenfalls in Gegenwart eines Verdünnungsmittels sowie gegebenenfalls in Gegenwart eines Phasentransfer-Katalysators umsetzt.

3) Halogenide der Formel (II)

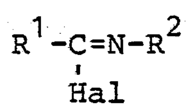

(II)

in welcher

$R^1$ für den Rest einer bei Pyrethroiden verwendbaren Säure steht,

$R^2$ für Alkoxy, Amino, Alkylamino, Dialkylamino oder einen über Stickstoff gebundenen stickstoffhaltigen Heterocyclus steht,

Hal für Halogene steht.

4) Verfahren zur Herstellung der Halogenide der Formel (II) gemäß Anspruch 3, indem man stickstoffsubstituierte Carbonsäureamide, für die die beiden tautomeren Formel (IVa) und (IVb) zur Beschreibung dienen können:

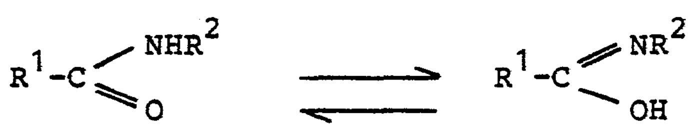

(IVa)
(IVb)

in welchen

$R^1$ und $R^2$ die obengenannten Bedeutungen besitzen,

mit bekannten Halogenierungsmitteln gegebenenfalls in Gegenwart eines Katalysators und gegebenenfalls in Gegenwart eines Säureakzeptors sowie gegebenenfalls in Gegenwart eines Verdünnungsmittels umsetzt.

5) N-substituierte Iminoester der Formel (I), in welcher $R^1$ für die Gruppierung

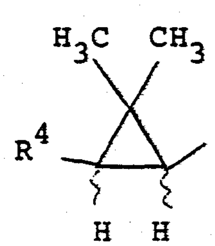

in welcher

$R^4$ für Alkyl, Alkenyl oder Alkinyl mit jeweils bis zu 6 C-Atomen, die gegebenenfalls einen carbocyclischen Ring enthalten und die gegebenenfalls substituiert sind durch ein oder mehrere Halogenatome, insbesondere Fluor, Chlor oder Brom, die außerdem gegebenenfalls substituiert sind durch einen Phenylrest, der gegebenenfalls durch Halogen, insbesondere Fluor, Chlor oder Brom substituiert ist, steht und

$R^5$ für Wasserstoff oder Methyl steht

oder für die Gruppierung

$$\begin{array}{c} \quad R^7 \\ \quad | \\ R^6-CH- \end{array}$$

in welcher

$R^6$ für Phenyl steht, das gegebenenfalls substituiert durch $C_1$-$C_6$-Alkyl, Halogenalkyl mit 1 bis 2 C-Atomen und bis zu 5 Halogenatomen, insbesondere durch Fluor, Chlor oder Brom, Halogenalkoxy mit 1 bis 2 C-Atomen und bis zu 5 Halogenatomen, insbesondere Fluor, Chlor oder Brom, Methylendioxy oder Ethylendioxy, die jeweils gegebenenfalls durch Halogen, insbesondere Fluor, Chlor oder Brom, substituiert sind und

$R^7$ für $C_2$-$C_4$-Alkyl, bevorzugt Isopropyl, steht,

$R^2$ für $C_1$-$C_4$-Alkoxy, Amino, $C_1$-$C_4$-Alkylamino, $C_1$-$C_4$-Dialkylamino oder einen über Stickstoff gebundenen Morpholino-, Pyrrolidino- oder Piperidino-Rest steht und

$R^3$ für Benzyl, das in $\alpha$-Stellung gegebenenfalls durch CN, $-C\equiv CH$, $-C=C=CH_2$, substituiert ist und das im Kern gegebenenfalls durch Halogen, insbesondere Fluor, Chlor oder Brom oder Phenoxy, Benzyl oder Benzyloxy, die gegebenenfalls durch Halogen, wie Fluor, Chlor oder Brom substituiert sind, steht.

6) Schädlingsbekämpfungsmittel, gekennzeichnet durch einen Gehalt an mindestens einem N-substituierten Iminoester der Formel (I).

7) Verwendung von N-substituiertem Iminoester der Formel (I) zur Bekämpfung von Schädlingen.

8) Verfahren zur Bekämpfung von Schädlingen, dadurch gekennzeichnet, daß man N-substituierte Iminoester der Formel (I) auf Schädlinge und/oder ihren Lebensraum einwirken läßt.

9) Verfahren zur Herstellung von Schädlingsbekämpfungsmitteln, dadurch gekennzeichnet, daß man N-substituierte Iminoester der Formel (I) mit Streckmitteln und/oder oberflächenaktiven Mitteln vermischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0095636

Nummer der Anmeldung

EP 83 10 4787

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US - A - 4 291 055 (CHEN)<br>* Patentansprüche *<br>---- | 1 | C 07 C 131/105<br>C 07 C 119/16<br>A 01 N 53/00<br>A 01 N 37/32<br>C 07 C 131/00 |

RECHERCHIERTE SACHGEBIETE (Int. Cl. 3)

C 07 C 131/00
C 07 C 119/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Die Definitionen von $R_1$ und $R_3$ im Anspruch 1 entsprichen nicht der Forderung nach Deutlichkeit des Artikels 84. Die Angabe "ein Rest eines bei Pyrethroiden verwendbaren Alkohols (und Säure) lässt nicht erkennen welche Verbindungen tatsächlich beantsprucht werden.

| Recherchenort | Abschlußdatum der Recherche | Prüfer |
|---|---|---|
| Den Haag | 19-08-1983 | GAUTIER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82